(19) Europäisches Patentamt

European Patent Office

Office européen des brevets

(11) **EP 0 595 110 B1**

(12) **EUROPÄISCHE PATENTSCHRIFT**

(45) Veröffentlichungstag und Bekanntmachung des Hinweises auf die Patenterteilung:
**07.01.1998 Patentblatt 1998/02**

(51) Int. Cl.$^6$: **A61K 9/28**

(21) Anmeldenummer: **93116525.2**

(22) Anmeldetag: **13.10.1993**

(54) **Hüllmaterial für orale Arzneiformen**

Coating material for orally administrable forms

Matériau d'enrobage pour formes d'administration orale

(84) Benannte Vertragsstaaten:
**AT BE CH DE DK ES FR GB GR IE IT LI LU NL PT SE**

(30) Priorität: **24.10.1992 DE 4236025**

(43) Veröffentlichungstag der Anmeldung:
**04.05.1994 Patentblatt 1994/18**

(73) Patentinhaber: **MERCK PATENT GmbH**
**64293 Darmstadt (DE)**

(72) Erfinder:
• **Siefke, Verena**
**D-77564 Offenburg (DE)**
• **Hanstein, Ullrich, Dr.**
**D-64367 Mühltal (DE)**
• **Weckenmann, Hans Peter, Dr.**
**D-64289 Darmstadt (DE)**
• **Bauer, Kurt, Prof.Dr.**
**D-79112 Freiburg (DE)**

(56) Entgegenhaltungen:
**EP-A- 0 485 840**

• **DATABASE WPI Week 8630, Derwent Publications Ltd., London, GB; AN 86-194734 & JP-A-61 129 138 (TAKEDA CHEMICAL IND KK) 17. Juni 1986**

Anmerkung:    Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Gegenstand der Erfindung ist eine neue orale Arzneiform, enthaltend wenigstens einen Wirkstoff und wenigstens ein den Wirkstoff umschließendes Hüllmaterial, das wenigstens einen in Wasser und den Verdauungssäften unlöslichen Filmbildner enthält, dadurch gekennzeichnet, daß das Hüllmaterial zusätzlich wenigstens ein Cyclodextrin und/oder wenigstens eines seiner Derivate enthält.

Ferner ist Gegenstand der Erfindung ein Hüllmaterial für orale Arzneiformen, enthaltend wenigstens einen in Wasser und den Verdauungssäften unlöslichen Filmbildner, dadurch gekennzeichnet, daß es zusätzlich wenigstens ein Cyclodextrin und/oder wenigstens eines seiner Derivate enthält.

Der Erfindung lag die Aufgabe zugrunde, orale Arzneiformen zu schaffen, bei denen die Wirkstoffe gezielt im Colon freigesetzt werden. Derartige Arzneiformen sind an sich bekannt.

In der Regel enthalten sie einen magen- und dünndarmresistenten Überzug, der erst im Colon abgebaut wird und dadurch die Freisetzung des Wirkstoffs ermöglicht. So ist in der EP 0 485 840 A2 ein Überzugsmittel beschrieben, daß ein im Colon abbaubares Polysaccharid und in Mischung damit ein filmbildendes Polymermaterial enthält. Derartige Überzugsmittel zeigen jedoch noch gewisse Nachteile. So sind sie spröde, und die Wirkstoffe werden nicht immer spezifisch im Colon freigesetzt.

In der DE 41 31 292 A1 werden Galactomannanderivate zur Umhüllung oder Einbettung von Arzneimittelwirkstoffen beschrieben. Bei diesen Galactomannanderivaten handelt es sich um neue Stoffe, deren toxikologische Unbedenklichkeit bisher noch nicht nachgewiesen ist.

In anderen Fällen, z.B. bei Mikrokapseln mit semipermeablen Umhüllungen, z.B. Ethylcellulose, unterliegt die Freisetzung großen individuellen Schwankungen.

Diese Nachteile der bekannten Arzneiformen werden durch die oben beschriebene orale Arzneiform vermieden oder wenigstens vermindert.

Als Arzneiformen eignen sich insbesondere Tabletten, Dragees, Sachets, Kapseln, Pellets, Streukügelchen, Granulate, Kristalle oder Pulver. Die Arzneiform kann auch in einer Einbettung bestehen, die wenigstens einen Wirkstoff enthält, welcher zusammen mit wenigstens einem Cyclodextrin in einem in den Verdauungssäften unlöslichen Filmbildner eingebettet ist.

Als Filmbildner eignen sich erfindungsgemäß bevorzugt Polyacrylate, Polymethacrylate sowie deren Mischpolymerisate sowie auch Ethylcellulosen, besonders hochsubstituierte Ethylcellulosen. Besonders vorteilhaft verwendet man handelsübliche Dispersionen, die Copolymerisate aus Acryl- und Methacrylsäureestern enthalten und einen geringen Gehalt an quartären Ammoniumgruppen aufweisen, wobei das molare Verhältnis dieser Ammoniumgruppen zu den übrigen neutralen (Meth)acrylsäureestern zwischen etwa 1:10 und 1:50, vorzugsweise 1:20 und 1:40 und das mittlere Molekulargewicht bei etwa 150 000 liegt.

Die erfindungsgemäß verwendeten Cyclodextrine sind $\alpha$-glykosidisch verknüpfte Oligosaccharide, im Gegensatz zu den $\beta$-glykosidisch verknüpften Polysacchariden, die gemäß EP 0 485 840 A2 verwendet werden.

Als Cyclodextrin ist $\beta$-Cyclodextrin ("CD"; sieben Glucoseeinheiten) bevorzugt. Es sind jedoch auch $\alpha$-Cyclodextrin (sechs Glucoseeinheiten) und $\gamma$-Cyclodextrin (acht Glucoseeinheiten) geeignet. Als Derivate der Cyclodextrine kommen in Betracht Hydroxypropyl-CD, Hydroxyethyl-CD, Poly-CD.

Das erfindungsgemäße Hüllmaterial kann weitere Hilfs- und/oder Zusatzstoffe enthalten. So ist der Zusatz von Weichmachern vorteilhaft. Als solche eignen sich besonders bevorzugt Alkylester von Di- oder Tricarbonsäuren wie Diethylphthalat ("DEP") oder Triethylcitrat ("TEC"). Wie man im Polarisationsmikroskop erkennen kann, liegt CD in den TEC enthaltenden Materialien im wesentlichen unverändert vor; bei den DEP enthaltenden Materialien ist dagegen eine kristalline Struktur zu erkennen. Weitere geeignete Weichmacher sind z.B. (andere) Citronen- und Weinsäureester (Acetyltriethyl-, Acetyltributyl-, Tributylcitrat); Glycerin und Glycerinester (Glycerindiacetat, -triacetat, acetylierte Monoglyceride, Rizinusöl); Phthalsäureester (Dibutyl-, Diamyl-, Dimethyl-, Dipropyl-, Di-(2-Methoxy- oder -Ethoxyethyl)-phthalat, Ethylphthalyl- und Butylphthalylethyl-und -butylglykolat); Alkohole (Propylenglykol, Polyethylenglykol verschiedener Kettenlängen); Adipate (Diethyl-, Di-(2-Methoxy- oder -Ethoxyethyl)-adipat); Benzophenon; Diethyl- und Dibutylsebacat, -succinat, -tartrat; Diethylenglykoldipropionat; Ethylenglykoldiacetat, -dibutyrat, -dipropionat; Tributylphosphat, Tributyrin; Polyethylenglykolsorbitanmonooleat; Sorbitanmonooleat; Polyethylenoxid-Polypropylenoxid-Blockpolymerisate.

Außerdem ist der Zusatz geringer Mengen wasserlöslicher Substanzen wie Polyethylenglykole, Polyvinylpyrrolidon, Copolymerisat aus Polyvinylpyrrolidon und Polyvinylacetat, Hydroxypropylcellulose, Hydroxypropylmethylcellulose möglich. Auch Feststoffe wie Talkum und/oder Magnesiumstearat sowie Farbstoffe, Pigmente können dem Hüllmaterial zugesetzt werden. Daneben kann ein Zusatz von lipophilen Substanzen, wie z.B. 20-30 % Stearinsäure, die Wasserdampfpermeabilität der Filme herabsetzen und somit die Haltbarkeit von feuchtigkeitsempfindlichen Stoffen verbessern.

Gegebenenfalls kann eine Isolierschicht zwischen Kern und Hilfsmaterial aufgetragen werden, die z.B. aus Hydroxypropylcellulose, Hydroxypropylmethylcellulose oder Polyvinylpyrrolidon bestehen kann.

Auch kann ein abschließender Schutzlack zur Anwendung kommen. Hierfür geeignet sind z.B. Celluloseacetatphthalat, Hydroxypropylmethylcellulosephthalat, Polyvinylacetatphthalat, Schellack, Hydroxypropylmethylcelluloseacetatsuccinat, Carboxymethylcellulose, Celluloseacetattrimellitat, Copolymere aus Maleinsäure- und Phthalsäurederivaten.

Das Hüllmaterial besteht zweckmäßig aus etwa 30-90 %, bevorzugt 40-75 % Filmbildner, 0-30 %, bevorzugt 8-15 % Weichmacher und 10-70, bevorzugt 12-50 % Cyclodextrin.

Die Umhüllung der Wirkstoffe oder der pharmazeutischen Zubereitungen, das heißt der Zubereitungen, worin die Wirkstoffe zusammen mit üblichen oder erforderlichen pharmazeutischen Hilfsstoffen eingearbeitet sind, erfolgt nach den in der pharmazeutischen Technologie bekannten Methoden, bzw. den üblichen Verfahren zum Überziehen von Arzneiformen.

Das Einbetten von therapeutischen Wirkstoffen erfolgt ebenfalls nach in der pharmazeutischen Technologie bekannten Methoden. Es wird hierbei anstatt der bisher üblichen plastischen oder schmelzbaren Einbettungsmaterialien, z.B. Wachse, hydriertes Ricinusöl, Kunststoffe, wie Celluloseether oder -ester, Poly(meth)acrylsäureester, das erfindungsgemäße Hüllmaterial verwendet. Hierbei können weiterhin übliche pharmazeutische Hilfs- bzw. Zusatzstoffe mitverwendet werden, beispielsweise Weichmacher, Aromastoffe, Süßmittel, Hilfsstoffe wie z.B. Talkum, Calciumcarbonat, Mannitol, Cellulosepulver, lösliche Farbstoffe und Pigmente.

Die Hilfsstoffe werden, falls überhaupt, der Umhüllungsmischung beispielsweise in Mengen von 10 bis zu 100 Gew.-%, vorzugsweise von 20 bis 40 Gew.-%, bezogen auf das Gewicht der verwendeten Cyclodextrine, zugesetzt.

Aromastoffe, Süßmittel und Farbstoffe können den Mischungen in geringen Mengen zugesetzt werden, beispielsweise von 0,001 % bis 2 %.

Nähere Angaben über die üblichen Hilfs- und Zusatzstoffe sind der Fachliteratur, beispielsweise der Monographie von J.H. Saunders und K.C. Frisch "High Polymers", Verlag Interscience Publishers, 1962 bzw. 1964, zu entnehmen.

Die Umhüllung erfolgt zweckmäßig durch Aufsprühen von Lösungen in organischen Lösungsmitteln oder Suspensionen bzw. Dispersionen der genannten Substanzen in organischen Lösungsmitteln oder Wasser, wobei noch weitere Hilfsstoffe zugesetzt sein können, wie z.B. oberflächenaktive Substanzen, Pigmente.

Das Aufsprühen erfolgt z.B. im Dragierkessel oder in perforierten Kesseln, oder im Luftsuspensions- bzw. Wirbelschichtverfahren (z.B. Glatt-Wirbelschichtanlage WSG5).

Die Umhüllung kann auch im Koazervationsverfahren erfolgen, wobei sogenannte Mikrokapseln oder Mikropartikel gebildet werden.

Die Umhüllung kann auch durch Koagulation wäßriger Dispersionen oder Suspensionen oberhalb der Mindestfilmbildungstemperatur der zuvor genannten Substanzen erfolgen, indem der Wirkstoff mit der Dispersion vermischt und das Wasser durch Trocknen entfernt wird.

Überzogene Wirkstoffteilchen und überzogene Granulate können zu Tabletten verpreßt werden, überzogene Pellets in Hartgelatinekapseln abgefüllt werden.

Beim Überziehen von Wirkstoffteilchen oder Granulaten, die Wirkstoffteilchen enthalten, wird üblicherweise mehr Umhüllungsmaterial eingesetzt als bei Pellets oder Tabletten, da die Oberfläche, die bedeckt werden muß, wesentlich größer ist als bei Pellets oder Tabletten.

Da Tabletten in der Regel größer als Pellets sind, ist die zu bedeckende Oberfläche bei Tabletten entsprechend kleiner. Auf 1 Gewichtsteil Wirkstoff oder Arzneizubereitung können z.B. 0,02 bis 1 Gewichtsteile Hüllmaterial verwendet werden. Bevorzugt ist ein Gewichtsverhältnis von 1 Teil Wirkstoff und 0,04 bis 0,7, insbesondere 0,05 bis 0,7 Gewichtsteilen Hüllmaterial, ganz besonders bevorzugt sind 0,1 bis 0,7 Gewichtsteile Hüllmaterial. Das Aufbringen des Hüllmaterials in Lösung, Suspension bzw. Dispersion erfolgt zweckmäßig bei erhöhter Temperatur, vorzugsweise im Luftstrom (Zulufttemperatur 60 bis 120°; Temperatur der Abluft bis 100°).

Bei Einbettungen werden auf 1 Gewichtsteil Wirkstoff beispielsweise 0,05 bis 5,0 Gewichtsteile Hüllmaterial, vorzugsweise 0,08 bis 3,0 Gewichtsteile, ganz besonders bevorzugt 0,1 bis 2,0 Gewichtsteile verwendet. Die Herstellung dieser Zubereitungen erfolgt zweckmäßig bei Temperaturen zwischen 10° und 100°.

Die Herstellung dieser Darreichungsformen kann z.B. erfolgen:

a) Durch Lösen oder Dispergieren der Wirkstoffe oder deren Salzen in dem erfindungsgemäßen Hüllmaterial oder Mischungen hiervon, auch unter Schmelzen der genannten Substanzen und anschließend Wiederabkühlen, Zerkleinern, eventuell Zufügen weiterer Substanzen wie z.B. wasserlöslichen oder in Wasser quellbaren Substanzen und Verpressung zu Tabletten. Das Abkühlen der Schmelze und Zerkleinern kann auch in einem Schritt zusammengefaßt werden, indem die Schmelze in kaltem Wasser dispergiert wird oder einer Sprüherstarrung unterworfen wird.

Als Quellstoffe kommen z.B. in Frage: Methylcellulose, Hydroxyethylcellulose, Hydroxypropylcellulose, Hydroxypropylmethylcellulose (Cellulosemischether mit Propoxy-, Ethoxy- und Methoxysubstituenten), Alginsäure und ihre Salze (Na-, Ca-Salz, auch Mischungen aus Natriumalginat und Calciumsalzen, z.B. $CaHPO_4$), Stärke, Carboxymethylstärke, Carboxymethylcellulose und deren Salze (z.B. Na-Salz), Gummi arabicum, Karaya-Gummi, Ghatti Gum, Agar-agar, Carrageen, Xanthan-Gummi, Propylenglykolalginat, Pektin, Traganth.

b) Durch Mischen der Wirkstoffe, mit dem erfindungsgemäßen Hüllmaterial und gegebenenfalls Quellstoffen oder Mischungen dieser Substanzen, auch unter Anwendung von Wärme, und beispielsweise Verpressen der Mischungen, eventuell nach Zusatz weiterer Hilfsstoffe, zu Tabletten oder Formung zu Pellets sowie Granulaten.

c) Durch Mischen der Wirkstoffe mit Lösungen des erfindungsgemäßen Hüllmaterials in organischen Lösungsmitteln, wie Ethanol, Ethylacetat, Aceton oder Isopropanol, eventuell Mischen mit Trägermaterialien wie Cellulosen, sowie nachfolgendes Abdampfen der Lösungsmittel und Vermischen der erhaltenen Wirkstoffeinbettung mit weiteren Hilfsstoffen und Verarbeitung zu Formkörpern, wie Tabletten, Granulaten oder Pellets.

d) Durch Anfeuchten einer Mischung der Wirkstoffe und des erfindungsgemäßen Hüllmaterials sowie gegebenenfalls den genannten Quellstoffen mit organischen Lösungsmitteln wie Ethanol, Ethylacetat, Aceton oder Isopropanol, eventuell unter Beifügung von Bindemitteln, wie Polyvinylpyrrolidon oder Copolymeren aus Polyvinylpyrrolidon und Polyvinylacetat, Granulieren der erhaltenen Mischung, nachfolgendes Trocknen, Zusatz von eventuellen weiteren Hilfsstoffen und beispielsweise Pressung der Mischung zu Tabletten.

Ganz allgemein erfolgt die Herstellung dieser Arzneimittelzubereitungen in an sich bekannter Weise, wobei neben dem erfindungsgemäßen Hüllmaterials die bekannten und üblichen pharmazeutischen Hilfsstoffe sowie sonstige übliche Träger- und Verdünnungsmittel verwendet werden können.

Als derartige Träger- und Verdünnungsmittel kommen zum Beispiel Stoffe in Frage, die in folgenden Literaturstellen als Hilfsstoffe für Pharmazie, Kosmetik und angrenzende Gebiete empfohlen bzw. angegeben sind: Ullmanns Encyklopädie der technischen Chemie, Band 4 (1953), Seite 1 bis 39; Journal of Pharmaceutical Sciences, Band 52 (1963), Seite 918 und ff., H.V. Czetsch-Lindenwald, Hilfsstoffe für Pharmazie und angrenzende Gebiete; Pharm. Ind., Heft 2, 1961, Seite 82 und ff.; Dr. H.P. Fiedler, Lexikon der Hilfsstoffe für Pharmazie, Kosmetik und angrenzende Gebiete, 2. Auflage, Editio Cantor, Aulendorf in Württemberg (1981).

Beispiele für übliche Hilfsstoffe, Trägerstoffe und Verdünnungsmittel sind Gelatine, natürliche Zucker, wie Rohrzucker oder Milchzucker, Lecithin, Pektin, Stärke (z.B. Maisstärke) sowie Stärkederivate, Galactomannane, Polyvinylpyrrolidon, Gelatine, Gummi arabicum, Alginsäure, Tylose, Talkum, Kieselsäure (z.B. kolloidale) bzw. hochdisperses $SiO_2$, Lävulose, Traganth, Natriumchlorid, Stearate, Magnesium- und Calicumsalze von Fettsäuren mit 12 bis 22 C-Atomen, insbesondere der gesättigten (z.B. Stearate), Polyethylenglykol mit einem mittleren Molekulargewicht zwischen 200 und 20 000, vorzugsweise zwischen 200 und 5 000, insbesondere zwischen 200 und 1 000, oder deren Gemische und/oder Polymerisate aus Vinylpyrrolidon und/oder Mischpolymerisate aus Vinylpyrrolidon und Vinylacetat, Ester von aliphatischen gesättigten oder ungesättigten Fettsäuren (2 bis 22 C-Atome, insbesondere 10 bis 18 C-Atome) mit einwertigen aliphatischen Alkoholen (1 bis 20 C-Atome) oder mehrwertigen Alkoholen wie Glykolen, Glycerin, Diethylenglykol, Pentaerythrit, Sorbit, Mannit usw. die gegebenenfalls auch verethert sein können, Benzylbenzoat, Dioxolane, Glycerinformale, Tetrahydrofurfurylalkohol, Polyglykolether mit $C_1$- bis $C_{12}$-Alkoholen, Dimethylacetamid, Lactamide, Lactate, Ethylcarbonate, Silicone (insbesondere mittelviskose Polydimethylsiloxane), Calciumcarbonat, Natriumcarbonat, Calciumphosphat, Natriumphosphat, Magnesiumcarbonat, Gummi arabicum, Alginsäure, Stearate, Fette und ähnlich wirkende Stoffe.

Daneben können die Darreichungsformen grenzflächenaktive Substanzen enthalten. Als Beispiele seien genannt: Alkaliseifen, wie Alkalisalze höherer Fettsäuren (z.B. Na-palmitat, Na-stearat) oder deren Derivate (z.B. Na-rizinolatschwefelsäureester); sulfurierte Verbindungen oder sulfonierte Verbindungen, die durch Umsetzung von höheren Fettalkoholen mit Schwefelsäure oder Chlorsulfonsäure entstehen und z.B. als Natriumsalze eingesetzt werden (z.B. Natriumlaurylsulfat, Natriumcetylsulfat, Natriumstearylsulfat, Natriumcetylsulfonat); Salze der Gallensäuren; Saponine; quartäre Ammoniumverbindungen; Partialfettsäureester des Sorbitans; Partialfettsäureester und Fettsäureester des Polyoxyethylensorbitans; Sorbitolether des Polyoxyethylens; Fettsäureester des Polyoxyethylen; Fettalkoholether des Polyoxyethylens; Fettsäureester der Saccharose; Fettsäureester des Polyglycerols; Proteine; Lecithine.

Die Darreichungsformen können auch Füllmittel enthalten, insbesondere wenn Komprimate hergestellt werden sollen. Als solche kommen in Frage:

Gereinigte Cellulose oder mikrokristalline Cellulose, Calciumhydrogenphosphat, Milchzucker, Stärken (z.B. Kartoffelstärke, Maisstärke), Glucose, Mannit und Saccharose, sowie Füllmittel mit Bindemittelfunktion, wie mikrokristalline Cellulose, hydrolysierte oder teilabgebaute Stärken und Mischkristallisate aus Cellulosepulver und Lactose.

Die Darreichungsformen können darüber hinaus Fließregulierungsmittel enthalten, wie z.B. hochdisperse Kieselsäuren. Außerdem kann der Einsatz von Formtrennmitteln in der Darreichungsform sinnvoll sein. Als solche wären zu nennen: Talk oder silikonisierter Talk, Calcium- und Magnesiumstearat, Stearinsäure, Paraffin, hydrierte Fette und Öle, Siliconölemulsionen. In der Regel ist jedoch ein Zusatz von Formtrennmitteln nicht nötig, da die Cyclodextrine selbst bereits Formtrennmittel-Charakter besitzen.

Als weitere Hilfsstoffe kommen auch Stoffe in Frage, die den Zerfall bewirken (Sprengmittel) wie quervernetztes Polyvinylpyrrolidon, Natriumcarboxymethylstärke, Natriumcarboxymethylcellulose, Formaldehydgelatine, Formaldehydcasein, Polyacrylsäure, Ultraamylopektin.

Darüber hinaus ist der Zusatz von Stabilisatoren, Farbstoffen, Antioxidantien und Komplexbildnern (z.B. Ethylendiamin - tetraessigsäure) sowie von Säuren wie Citronensäure, Weinsäure, Maleinsäure, Fumarsäure, möglich.

Als Antioxidantien kommen beispielsweise Natriummetabisulfit, Cystein, Ascorbinsäure und deren Ester (z.B. -palmitat), Flavanoide, Gallussäure-alkylester, Butylhydroxyanisol, Nordihydroguajaretsäure, Tocopherole sowie Tocopherole + Synergisten (Stoffe, die Schwermetalle durch Komplexbildung binden, beispielsweise Lecithin, Ascorbinsäure, Citronensäure, Phosphorsäure) zur Anwendung.

Als Konservierungsmittel kommen beispielsweise Sorbinsäure, p-Hydroxybenzoesäureester (z.B. Niederalkylester), Benzoesäure, Natriumbenzoat, Trichlorisobutylalkohol, Phenol, Kresol, Benzethoniumchlorid und Formalinderivate in Betracht.

Zum Aufbringen des erfindungsgemäßen Hüllmaterials können Lösungsmittel verwendet werden aus der Gruppe der wäßrigen Lösungsmittel, Alkohole, Ketone, Ester, Ether, aliphatischen Kohlenwasserstoffe, halogenierten Kohlenwasserstoffe, cycloaliphatischen, heterocyclischen Lösungsmittel und deren Gemische. Typische Lösungsmittel sind unter anderem Aceton, Diaceton- alkohol, Methanol, Ethanol, Isopropylalkohol, Butylalkohol, Methylacetat, Ethylacetat, Isopropylacetat, n-Butylacetat, Methylisobutyl-keton, Methyl-propyl-keton, n-Hexan, n-Heptan, Ethylglykol-monoethylether, Ethylenglykolmonoethylacetat, Dichlormethan, 1,2-Dichlorethan, 1,2- oder 1,3-Dichlorpropan, Tetrachlormethan, Nitroethan, Nitropropan, Tetrachlorethan, Cyclohexan, Cyclooctan, Benzol, Toluol, Naphtha, 1,4-Dioxan, Tetrahydrofuran, Diethylenglykoldimethylether, Wasser und deren Gemische wie Aceton und Wasser, Aceton und Methanol, Aceton und Ethanol, Dichlormethan und Methanol sowie 1,2-Dichlorethan und Methanol. Im Laufe des Umhüllungsprozesses werden diese Lösungsmittel wieder entfernt.

Als Wirkstoffe, die mit dem erfindungsgemäßen Hüllmaterial formuliert werden können, kommen alle diejenigen in Betracht, die oral verabfolgt werden können und deren Freigabe erst im Colon erwünscht sein kann. Dazu gehören z.B. Darm-Mittel wie Mesalazin (5-Aminosalicylsäure) oder Laxantien wie Bisacodyl, weiterhin Peptide, Herz-Kreislauf-Therapeutika, Antirheumatika/Analgetika, Mittel zur Therapie von Dickdarmerkrankungen (Morbus Crohn, Colitis ulcerosa), Antiasthmatika, Antifibrinolytika, Antihämorrhagika, Antitumormittel, Enzympräparate, Antibiotika, Antimykotika, Substanzen mit Wirkung auf das ZNS (Zentralnervensystem).

Eine wichtige Klasse von Wirkstoffen, die erst im Colon freigesetzt werden sollen, sind solche mit Peptid- oder Proteinstruktur, z.B. Insulin. Sie würden in den oberen Darmabschnitten durch die körpereigenen proteolytischen Enzyme abgebaut, bevor sie zur Wirkung kommen können; im Colon ist der Gehalt an proteolytischen Enzymen dagegen so gering, daß eine ausreichende Einwirkungs- bzw. Resorptionszeit verbleibt.

Beispiele für Peptid-Wirkstoffe sind insbesondere: ACTH (Adrenocorticotropes Hormon), Corticostatin, Calcitonin, Insulin, Oxytocin, Somatostatin und Analoga, LHRH-Analoga, Bombesin-Analoga, Cholecystokinin und Derivate, Endothelin und Analoga, Thrombin-Inhibitoren, Peptide Growth Factors (z.B. IGF, EGF, NGF), Magainine (PGS peptides), Gastrin-Analoga, Parathormon-Analoga, Neurokinin und Analoga, VIP (Vasoactive intestinal polypeptide) und Analoga, ANP (Atriales Natriuretisches Peptid) und Analoga, Neokyotrophin und Analoga, Angiotensin-Analoga, Enkephaline, Dynorphine, Dermorphine, Deltorphine, Renin-inhibierende Peptide, Tumor-Growth-Factor-Peptide, MSH (Melanocyte Stimulating Hormone)-Analoga, Mitotoxine, Tyrphostine, Chromogranin A, Thymopentin, TRH (Thyrotropine releasing hormone) und Analoga, Substanz-P, Tuftsin, Fibronectin und peptidische Immunmodulatoren wie Cyclosporin A, FK 506 und Neuropeptid Y.

Das erfindungsgemäße Hüllmaterial ist in künstlichem Dünndarmsaft nahezu undurchlässig. Nach Inkubation im "colonic microflora test" ("CMT"; vgl. Dissertation C. Wohlschlegel, Freiburg, 1990) findet dagegen ein enzymatischer Abbau von CD statt, durch den die Filme porös und damit durchlässig werden, so daß die umhüllten Wirkstoffe freigesetzt und im Colon wirksam werden können.

Ein besonderer Vorteil des neuen Hüllmaterials liegt darin, daß seine Bestandteile bekannt und physiologisch völlig unbedenklich sind.

Vor und nachstehend bedeuten alle Prozentangaben Gewichtsprozente. Temperaturen sind in °C angegeben.

**Beispiel 1: Hüllmaterial (Film)**

**1.1 Herstellung**

Zu 66,6 g einer 30%igen wässerigen Dispersion (Handelspräparat) eines Copolymerisates aus 30 Teilen Ethylacrylat, 65 Teilen Methylmethacrylat und 5 Teilen Trimethylammonioethylmethacrylat-chlorid gibt man innerhalb 2 Min. unter Rühren (180 U/Min.) ein Gemisch von 4 g DEP und einer Dispersion von 4 g CD in 27,3 ml Wasser hinzu und rührt noch 10 Min. bei Raumtemperatur.

Zur Filmcharakterisierung wird aus der so erhaltenen Dispersion mit dem Erichsen-Filmziehgerät Modell 509/1 bei 40° und einer Ziehgeschwindigkeit von 12 mm/s ein Film auf Polyesterfolien gezogen. Es wird ein Rakel mit einer Spalthöhe von 200 $\mu$m verwendet. Die Verfilmung dauert ca. 30 Minuten, anschließend wird der Film bei 20-25° auf der Folie gelagert und zur eigentlichen Charakterisierung wieder von der Trägerfolie gelöst.

Analog werden Dispersionen folgender Zusammensetzungen erhalten (in %):

| Nr. | Filmbildner | Weichmacher | | CD | Wasser |
|---|---|---|---|---|---|
| | | TEC | DEP | | |
| 1 | 19,23 | 3,85 | 0 | 3,85 | 73,07 |
| 2 | 19,23 | 3,85 | 0 | 5,77 | 71,15 |
| 3 | 19,23 | 3,85 | 0 | 7,69 | 69,23 |
| 4 | 19,23 | 0 | 3,85 | 3,85 | 73,07 |
| 5 | 19,23 | 0 | 3,85 | 5,77 | 71,15 |
| 6 | 19,23 | 0 | 3,85 | 7,69 | 69,23 |
| 7 | 19,23 | 0 | 3,85 | 9,62 | 67,30 |
| 8 | 17,24 | 0 | 3,45 | 10,34 | 68,97 |
| 9 | 16,95 | 0 | 3,39 | 11,86 | 67,80 |
| 10 | 16,66 | 0 | 3,33 | 13,33 | 66,68 |
| 11 | 16,39 | 0 | 3,28 | 14,75 | 65,58 |
| 12 | 16,13 | 0 | 3,23 | 16,13 | 64,51 |
| 13 | 15,63 | 0 | 3,13 | 18,75 | 62,49 |

## 1.2 Filmcharakterisierung

### 1.2.1 Filmdickenbestimmung

Die Filmdicke wird durch ein magnetinduktives Verfahren bestimmt (Minitest 3000, Fa. Erichsen). Es finden Messungen an sechs verschiedenen Punkten statt, die Ergebnisse werden gemittelt. Die Filmdicken liegen zwischen 35 und 70 $\mu$m.

### 1.2.2 Permeabilität in künstlichem Dünndarmsaft

Die Überprüfung der Filme hinsichtlich ihrer Permeabilität erfolgt mit Hilfe der Franzschen Zellen (vgl. C.L. Gummer et al., Int. J. Pharm. 40 (1987) 101-104). Hierzu wird ein Stück Film zwischen Donor und Akzeptor gespannt. Das Akzeptor-Gefäß wird mit künstlichem Dünndarmsaft (Phosphatpuffer pH 6,8 R, DAB 10) gefüllt und auf 37° temperiert. Mit einem Magnetrührer wird die Flüssigkeit durchmischt. Im Donor befindet sich eine konz. Lösung von Mesalazin (5 $\mu$g/ml) in Phosphatpuffer pH 6,8. Der Arzneistoff dient als Indikatorsubstanz für die Permeabilität des Films. Nach 2 h, 4 h und 6 h erfolgt eine Probenentnahme von 2 ml aus dem Akzeptor, die durch Phosphatpuffer ersetzt wird. Die Probe wird photometrisch vermessen (Uvikon 820, Wellenlänge 330 nm), die Nachweisgrenze liegt bei 2 $\mu$g/ml. Mit Hilfe der ermittelten Mesalazin-Konzentration im Akzeptor wird die Permeabilität der Filme bestimmt. Alle in Abschnitt 1.1 genannten Filme waren unter diesen Bedingungen bis zu 6 Std. praktisch undurchlässig.

### 1.2.3 Abbaubarkeit im CMT (Colonic Microflora Test)

Zur Überprüfung der Abbaubarkeit im Dickdarm wird der CMT (vgl. Pharm. Pharmacol. Lett. (1992) 2, 62-65) herangezogen. Dies ist ein Gemisch aus Schweinecaecum, Ausscheidungssekret von Ileostomiepatienten und Phosphatpuffer pH 6,4 R, DAB 10, (5:5:1). Die Mischung wird unter anaeroben Bedingungen, d.h. unter $N_2/CO_2$-Begasung im Verhältnis 5:1, bei 37° inkubiert.

Es werden runde Filmstücke mit einem Radius von ca. 0,7 cm mit dem Ileostomie-Schweinecaecum-Gemisch definierte Zeiten (zwischen 2 und 6 Std.) lang inkubiert, anschließend mit Wasser gespült und bei Raumtemperatur getrocknet. Zur Probenvorbereitung werden die Filmstücke dann auf einen Objektträger aufgeklebt und mit Gold bedampft. Die Oberfläche der Filme wird rasterelektronenmikroskopisch untersucht. Es zeigt sich eine deutlich poröse Struktur im Gegensatz zu Filmproben, die nicht mit CMT behandelt werden.

**Beispiel 2: Hüllmaterial (Film)**

Zu 50 g einer 25%igen wässerigen Dispersion (Handelspräparat) einer Ethylcellulose gibt man innerhalb 2 Min. unter Rühren (180 U/Min.) ein Gemisch von 1,2 g DEP und einer Dispersion von 4,75 g CD in 36,3 g Wasser hinzu und rührt noch 15 Min. bei Raumtemperatur.

Analog werden Dispersionen folgender Zusammensetzungen erhalten (in %):

| Nr. | Filmbildner | Weichmacher | | | CD | Wasser |
|-----|-------------|------|------|------|------|--------|
|     |             | TEC  | DEP  | DBS*) |      |        |
| 1   | 11,93       | 1,45 | 0    | 0    | 3,44 | 83,18  |
| 2   | 10,70       | 1,30 | 0    | 0    | 5,15 | 82,85  |
| 3   | 11,93       | 0    | 1,45 | 0    | 3,44 | 83,18  |
| 4   | 10,70       | 0    | 1,30 | 0    | 5,15 | 82,85  |
| 5   | 11,93       | 0    | 0    | 1,45 | 3,44 | 83,18  |
| 6   | 10,70       | 0    | 0    | 1,30 | 5,15 | 82,85  |
| DBS = Dibutylsebacat | | | | | | |

**Beispiel 3: Überzogene Tabletten**

**3.1 Herstellung**

Man mischt 330 g des Handelspräparats gemäß Beispiel 1.1 mit einer Suspension von 20 g TEC und 30 g CD in 370 ml Wasser durch Rühren mit einem Flügelrührer und rührt noch weitere 10 Minuten.

Mit Hilfe dieser Dispersion werden 1,5 kg Tablettenkerne folgender Zusammensetzung besprüht:

| | |
|---|---|
| Mesalazin | 100 mg |
| mikrokristalline Cellulose | 35 mg |
| Lactose | 35 mg |
| Polyvinylpyrrolidon | 7 mg |
| Maisstärke | 20 mg |
| hochdisperse Kieselsäure | 3,6 mg |
| Carboxymethylcellulose, Na-Salz | 1,8 mg |
| Magnesiumstearat | 1,8 mg |
| | 204,2 mg |
| (Durchmesser 8 mm; Höhe 3,76 mm; Oberfläche 1,96 cm$^2$). | |

Während des Sprühvorganges wird die Dispersion mit einem Flügelrührer gerührt (kontinuierliche Sprühweise, Sprühluft 3 bar; Durchfluß 10 g. Min.$^{-1;}$ Düsendurchmesser 0,6 mm; Temperatur: Eingang 63°, Kernbett 33°, Ausgang 44°; Sprühdauer ca. 1 Std.). Die erhaltenen mit je 17,6 mg Hüllmaterial überzogenen Tabletten (Einzelgewicht 221,8 mg) werden über Nacht bei 40° getrocknet.

**3.2 Tablettencharakterisierung**

**3.2.1 Beständigkeit im künstlichen Dickdarm**

Zur Überprüfung der Tabletten hinsichtlich ihrer Beständigkeit im künstlichen Dünndarm werden sie 6 Std. im Zerfallstester bewegt und anschließend optisch überprüft. Außerdem wird der Gehalt von Mesalazin im Prüfmedium bestimt. Alle Tabletten waren unter diesen Bedingungen mindestens 6 Std. stabil.

### 3.2.2 Abbaubarkeit im CMT

Die Tabletten werden im CMT für 4 Std., 6 Std. und 24 Std. inkubiert und anschließend ihr Freisetzungsverhalten in einer Paddle-Apparatur bestimmt. Hierbei läßt sich erkennen, daß die Permeabilität der Tabletten mit höherem Anteil an β-CD und längerer Inkubationszeit steigt.

### Beispiel 4: Überzogene Tabletten

Man mischt 500 g des Handelspräparates gemäß Beispiel 2 mit einer Suspension von 12 g TEC und 47,5 g CD in 670,5 ml Wasser durch Rühren mit einem Flügelrührer und rührt noch weitere 10 Minuten. Man verfährt wie in Beispiel 3 angegeben weiter.

### Patentansprüche

1. Orale Arzneiform, enthaltend wenigstens einen Wirkstoff und wenigstens ein den Wirkstoff umschließendes Hüllmaterial, das wenigstens einen in Wasser und den Verdauungssäften unlöslichen Filmbildner enthält, dadurch gekennzeichnet, daß das Hüllmaterial zusätzlich wenigstens ein Cyclodextrin - aus α-glykosidisch verknüpften Oligosacchariden - und/oder wenigstens eines seiner Derivate, sowie einen Weichmacher enthält.

2. Orale Arzneiform nach Anspruch 1, enthaltend wenigstens einen Wirkstoff und wenigstens ein den Wirkstoff umschließendes Hüllmaterial, das wenigstens einen in Wasser und den Verdauungssäften unlöslichen Filmbildner enthält, dadurch gekennzeichnet, daß das Hüllmaterial zusätzlich wenigstens ein Cyclodextrin - aus α-glykosidisch verknüpften Oligosacchariden - und/oder wenigstens eines seiner Derivate, sowie Diethylphthalat, Triethylcitrat oder Dibutylsebacat enthält.

3. Hüllmaterial für orale Arzneiformen, enthaltend wenigstens einen in Wasser und den Verdauungssäften unlöslichen Filmbilder, dadurch gekennzeichnet, daß es zusätzlich wenigstens ein Cyclodestrin - aus α-glykosidisch verknüpften Oligosacchariden - und/oder wenigstens eines seiner Derivate, sowie einen Weichmacher enthält.

4. Hüllmaterial für orale Arzneiformen nach Anspruch 3, enthaltend wenigstens einen in Wasser und den Verdauungssäften unlöslichen Filmbilder, dadurch gekennzeichnet, daß es zusätzlich wenigstens ein Cyclodextrin - aus α-glykosidisch verknüpften Oligosacchariden - und/oder wenigstens eines seiner Derivate, sowie Diethylphthalat, Triethylcitrat oder Dibutylsebacat enthält.

### Claims

1. Oral drug form comprising at least one active compound and at least one coating material which encloses the active compound and comprises at least one film-forming agent which is insoluble in water and digestive juices, characterized in that the coating material additionally comprises at least one cyclodextrin - from α-glycosidically linked oligosaccharides - and/or at least one of its derivatives, as well as a plasticizer.

2. Oral drug form according to Claim 1, comprising at least one active compound and at least one coating material which encloses the active compound and comprises at least one film-forming agent which is insoluble in water and digestive juices, characterized in that the coating material additionally comprises at least one cyclodextrin - from α-glycosidically linked oligosaccharides - and/or at least one of its derivatives, as well as diethyl phthalate, triethyl citrate or dibutyl sebacate.

3. Coating material for oral drug forms, comprising at least one film-forming agent which is insoluble in water and digestive juices, characterized in that it additionally comprises at least one cyclodextrin - from α-glycosidically linked oligosaccharides - and/or at least one of its derivatives, as well as a plasticizer.

4. Coating material for oral drug forms according to Claim 3, comprising at least one film-forming agent which is insoluble in water and digestive juices, characterized in that it additionally comprises at least one cyclodextrin - from α-glycosidlcally linked oligosaccharides - and/or at least one of its derivatives, as well as diethyl phthalate, triethyl citrate or dibutyl sebacate.

**Revendications**

1. Forme médicamenteuse orale, contenant au moins un principe actif et au moins un matériau d'enrobage qui entoure le principe actif, contenant au moins un agent filmogène insoluble dans l'eau et dans les sucs digestifs, caractérisée en ce que le matériau d'enrobage contient en outre au moins une cyclodextrine - provenant d'oligo-saccharides à liaison $\alpha$-glycosidique - et/ou au moins l'un de ses dérivés, ainsi qu'un plastifiant.

2. Forme médicamenteuse orale selon la revendication 1, contenant au moins un principe actif et au moins un matériau d'enrobage qui entoure le principe actif, contenant au moins un agent filmogène insoluble dans l'eau et dans les sucs digestifs, caractérisée en ce que le matériau d'enrobage contient en outre au moins une cyclodextrine - provenant d'oligosaccharides à liaison $\alpha$-glycosidique - et/ou au moins l'un de ses dérivés, ainsi que du phtalate de diéthyle, du citrate de triéthyle ou du sébaçate de dibutyle.

3. Matériau d'enrobage pour formes médicamenteuses orales, contenant au moins un agent filmogène insoluble dans l'eau et dans les sucs gastriques, caractérisé en ce qu'il contient en outre au moins une cyclodextrine - provenant d'oligosaccharides liés par liaison $\alpha$-glycosidique - et/ou l'un de ses dérivés, ainsi qu'un plastifiant.

4. Matériau d'enrobage pour formes médicamenteuses orales selon la revendication 3, contenant au moins un agent filmogène insoluble dans l'eau et dans les sucs gastriques, caractérisé en ce qu'il contient en outre au moins une cyclodextrine - provenant d'oligosaccharides liés par liaison $\alpha$-glycosidique - et/ou l'un de ses dérivés, ainsi que du phtalate de diéthyle, du citrate de triéthyle ou du sébaçate de dibutyle.